# EUROPEAN PATENT APPLICATION

(11) **EP 4 094 603 A1**
(43) Date of publication of application: **30.11.2022**
(21) Application number: 20936299.5
(22) Date of filing: 19.05.2020
(51) Int. Cl.: A24F 40/51

(54) **INHALATION DEVICE, INFORMATION PROCESSING DEVICE, AND CONTROL METHOD**

(71) Applicant: Japan Tobacco Inc., Tokyo 105-6927 (JP)
(72) Inventor: SERITA, Kazutoshi, Tokyo 130-8603 (JP); SUGANO, Yuka, Tokyo 130-8603 (JP); SENJU, Masatoshi, Tokyo 130-8603 (JP)
(74) Representative: Hoffmann Eitle
(86) International application number: PCT/JP2020/019703
(87) International publication number: WO 2021/234801

(57) **Abstract**

[Problem] To provide an assembly that can suitably detect biometric information of a user who uses an inhalation device.

[Solution] An inhalation device comprising: a first detection part that detects biometric information of a user; and a control part that controls the first detection part such that the first detection part detects first biometric information at a first timing, and detects second biometric information at a second timing that is later than the first timing.

## Description

### Technical Field

The present invention relates to an inhaler device, an information processing device, and a control method.

### Background Art

Inhaler devices such as electronic cigarettes and nebulizers that generate a material to be inhaled by a user have become widely popular. For example, an inhaler device generates an aerosol to which a flavor component has been imparted by using a substrate including an aerosol source for generating an aerosol and a flavor source for imparting a flavor component to the generated aerosol. A user can taste flavor by inhaling (hereinafter also referred to as a puff) the aerosol to which the flavor component has been imparted thus generated by the inhaler device.

In recent years, detecting biological information of a user using an inhaler device and using the biological information for various kinds of services are under consideration. For example, Patent Literature 1 discloses a technique for detecting biological information of a user from a portion of an inhaler device with which a finger of the user is in contact.

### Citation List

### Patent Literature

Patent Literature 1: International Publication No. 2019/175810

### Summary of Invention

### Technical Problem

However, a technique for detecting biological information of a user using an inhaler device is a recently developed technique and needs further improvement.

The present invention was accomplished in view of the above problem, and an object of the present invention is to provide a mechanism capable of properly detecting biological information of a user using an inhaler device.

### Solution to Problem

In order to solve the above problem, an aspect of the present invention provides an inhaler device including a first detector that detects biological information of a user; and a controller that controls the first detector to detect first biological information at a first timing and detect second biological information at a second timing later than the first timing.

The first detector may receive a reflected wave reflected by a body of the user and detect the biological information on a basis of the received reflected wave.

The first detector may transmit a transmission wave; and the reflected wave may be the transmission wave reflected by the body of the user.

The transmission wave may include at least one of a radio wave, an infrared ray, and/or light.

The controller may control processing for outputting first notification information, which is information concerning the first biological information and the second biological information.

The controller may determine whether or not to output the first notification information on the basis of the first biological information and the second biological information.

The controller may determine whether or not to output the first notification information by comparing the first biological information and the second biological information.

The inhaler device may further include a wireless communicator that performs wireless communication with another device, and the controller may control the wireless communicator to transmit the first notification information as controlling processing for outputting the first notification information.

The first notification information may further include identification information of a substrate used to generate a material to be inhaled by the user.

The inhaler device may further include a second detector that detects information concerning the inhaler device, and the controller may control the first detector to detect the first biological information while using, as the first timing, a timing at which the second detector detects input of a predetermined user's operation to the inhaler device, a user's action of inhaling a material generated by the inhaler device, setting of a substrate for generating the material in the inhaler device, or identification information of the substrate set in the inhaler device.

The controller may control the first detector to detect the first biological information while using, as the first timing, a timing at which a user's action of inhaling the material is detected by the second detector for a first time after a period for which the user's action of inhaling the material is not detected exceeds a predetermined period.

The controller may control the first detector to detect the second biological information while using, as the second timing, a timing after start of heating of the substrate set in the inhaler device.

The controller may control the first detector to detect the second biological information while using, as the second timing, a timing at which cancellation of setting of the substrate in the inhaler device is detected by the second detector or a timing at which a user's action of inhaling the material is detected by the second detector last time in a case where a period for which the user's action of inhaling the material is not detected exceeds a predetermined period.

The controller may control the first detector to detect the second biological information while using, as the second timing, a timing at which the second detector detects that a predetermined time has elapsed or the number of times of user's inhalation of the material has reached a predetermined number after start of heating of the substrate set in the inhaler device.

The controller may limit a function of generating a material to be inhaled by the user on the basis of the first biological information and the second biological information.

The biological information may include at least one of a blood pressure, a heart rate, a blood oxygen level, and/or a degree of oxygen saturation.

In order to solve the above problem, another aspect of the present invention provides an information processing device including a communicator that communicates with another device; and a controller that controls processing for outputting second notification information on the basis of first notification information received by the communicator, which is information concerning first biological information detected at a first timing by an inhaler device having a first detector that detects biological information of a user and second biological information detected at a second timing later than the first timing by the inhaler device.

The controller may control the communicator to transmit the second notification information as controlling processing for outputting the second notification information.

The controller may control the communicator to transmit, as the second notification information, information concerning an instruction to limit a function of generating a material to be inhaled by the user in the inhaler device.

The first notification information may further include identification information of a substrate used to generate a material to be inhaled by the user; and the information processing device may further include a memory that stores therein the first notification information.

The controller may control processing for outputting, as the second notification information, information indicative of the substrate recommended for the user on the basis of the first notification information stored in the memory.

In order to solve the above problem, another aspect of the present invention provides a control method for controlling an inhaler device including a first detector that detects biological information of a user, the control method including controlling the first detector to detect first biological information at a first timing and detect second biological information at a second timing later than the first timing.

### Advantageous Effects of Invention

According to the present invention, a mechanism that makes it possible to properly detect biological information of a user of an inhaler device is provided.

### Brief Description of Drawings

[Fig. 1] Fig. 1 is a schematic diagram of an inhaler device according to a first configuration example.
[Fig. 2] Fig. 2 is a schematic diagram of an inhaler device according to a second configuration example.
[Fig. 3] Fig. 3 is a block diagram illustrating an example of a configuration of a system according to an embodiment of the present invention.
[Fig. 4] Fig. 4 is a sequence diagram illustrating an example of a flow of processing performed in the system according to the present embodiment.

### Description of Embodiments

A preferred embodiment of the present invention is described in detail below with reference to the attached drawings. Note that in the specification and the drawings, structural elements having substantially identical functional configurations are given identical reference signs, and repeated description thereof is omitted.

### <<1. Configuration example of inhaler device>>

An inhaler device generates material to be inhaled by a user. In the example described below, the material generated by the inhaler device is an aerosol. Alternatively, the material generated by the inhaler device may be gas.

### (1) First configuration example

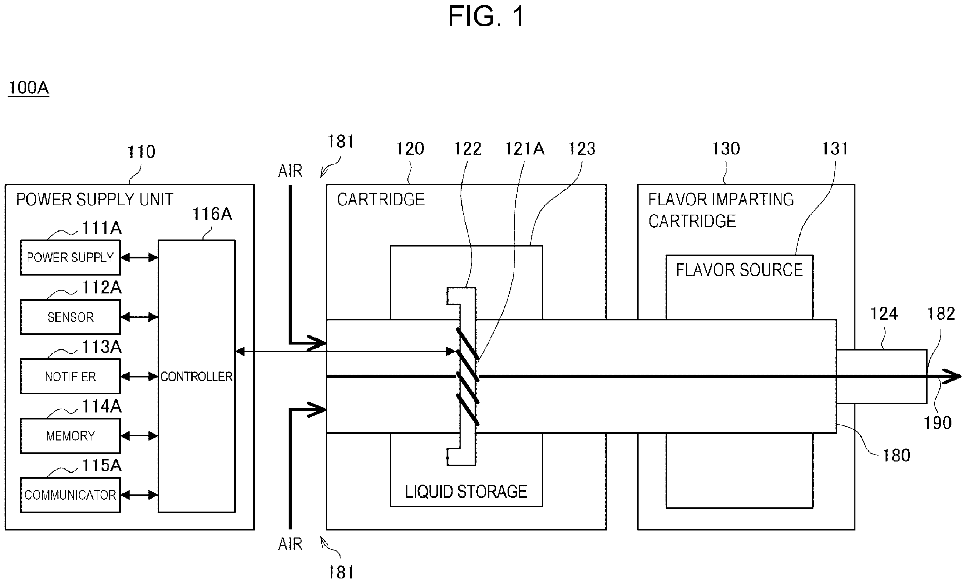

Fig. 1 is a schematic diagram of the inhaler device according to the first configuration example. As illustrated in Fig. 1, an inhaler device 100A according to the present configuration example includes a power supply unit 110, a cartridge 120, and a flavor imparting cartridge 130. The power supply unit 110 includes a power supply 111A, a sensor 112A, a notifier 113A, a memory 114A, a communicator 115A, and a controller 116A. The cartridge 120 includes a heater 121A, a liquid guide 122, and a liquid storage 123. The flavor imparting cartridge 130 includes a flavor source 131 and a mouthpiece 124. In the cartridge 120 and the flavor imparting cartridge 130, an airflow path 180 is defined.

The power supply 111A stores electric power. The power supply 111A supplies electric power to the structural elements of the inhaler device 100A under the control of the controller 116A. The power supply 111A may be a rechargeable battery such as a lithium ion secondary battery.

The sensor 112A acquires various items of information regarding the inhaler device 100A. In an example, the sensor 112A may be a pressure sensor such as a condenser microphone, a flow sensor, or a temperature sensor, and acquire a value generated in accordance with the user's inhalation. In another example, the sensor 112A may be an input device that receives information input by the user, such as a button or a switch.

The notifier 113A provides information to the user. The notifier 113A may be a light-emitting device that emits light, a display device that displays an image, a sound output device that outputs sound, or a vibration device that vibrates.

The memory 114A stores various items of information for operation of the inhaler device 100A. The memory 114A may be a non-volatile storage medium such as flash memory.

The communicator 115A is a communication interface capable of communication in conformity with any wired or wireless communication standard. Such a communication standard may be, for example, Wi-Fi (registered trademark) or Bluetooth (registered trademark).

The controller 116A functions as an arithmetic processing unit and a control circuit, and controls the overall operations of the inhaler device 100A in accordance with various programs. The controller 116A includes an electronic circuit such as a central processing unit (CPU) or a microprocessor, for example.

The liquid storage 123 stores an aerosol source. The aerosol source is atomized to generate an aerosol. The aerosol source is a liquid such as polyhydric alcohol or water. Examples of the polyhydric alcohol include glycerine and propylene glycol. The aerosol source may include a flavor component that is either derived from tobacco or not derived from tobacco. For the inhaler device 100A that is a medical inhaler such as a nebulizer, the aerosol source may include a medicine.

The liquid guide 122 guides, from the liquid storage 123, the aerosol source that is the liquid stored in the liquid storage 123, and holds the aerosol source. The liquid guide 122 is, for example, a wick formed by twining fiber material such as glass fiber or porous material such as porous ceramic. In this case, the capillary action of the wick guides the aerosol source stored in the liquid storage 123.

The heater 121A heats the aerosol source to atomize the aerosol source and generate the aerosol. In the example illustrated in Fig. 1, the heater 121A includes a coil wound around the liquid guide 122. When the heater 121A produces heat, the aerosol source held by the liquid guide 122 is heated and atomized to generate the aerosol. The heater 121A produces heat when receiving electric power from the power supply 111A. In an example, the electric power may be supplied in response to the sensor 112A detecting a start of the user's inhalation and/or an input of predetermined information. Subsequently, the supply of the electric power may be stopped in response to the sensor 112A detecting an end of the user's inhalation and/or an input of predetermined information.

The flavor source 131 is a structural element for imparting a flavor component to the aerosol. The flavor source 131 may include a flavor component that is either derived from tobacco or not derived from tobacco.

The airflow path 180 is a flow path of air to be inhaled by the user. The airflow path 180 has a tubular structure having an air inlet hole 181 and an air outlet hole 182 at both ends. The air inlet hole 181 is an inlet of air into the airflow path 180, and the air outlet hole 182 is an outlet of the air from the airflow path 180. The liquid guide 122 is on the airflow path 180 at an upstream position (closer to the air inlet hole 181), and the flavor source 131 is on the airflow path 180 at a downstream position (closer to the air outlet hole 182). Air flowing in through the air inlet hole 181 when the user inhales mixes with the aerosol generated by the heater 121A. Subsequently, as indicated by an arrow 190, the mixture fluid of the aerosol and the air passes through the flavor source 131 and is conveyed to the air outlet hole 182. When the mixture fluid of the aerosol and the air passes through the flavor source 131, the flavor component included in the flavor source 131 is imparted to the aerosol.

The mouthpiece 124 is to be held in a mouth of the user during inhalation. The mouthpiece 124 has the air outlet hole 182. When the user inhales with the mouthpiece 124 in his/her mouth, the mixture fluid of the aerosol and the air enters the oral cavity of the user.

The configuration example of the inhaler device 100A has been described above. The inhaler device 100A is not limited to the above configuration, and may be configured in various ways as exemplified below.

In an example, the inhaler device 100A does not have to include the flavor imparting cartridge 130. In this case, the cartridge 120 includes the mouthpiece 124.

In another example, the inhaler device 100A may include various types of aerosol sources. Still another type of aerosol may be generated by mixing a plurality of types of aerosols generated from the plurality of types of aerosol sources in the airflow path 180 and causing a chemical reaction.

In addition, means for atomizing the aerosol source is not limited to heating by the heater 121A. For example, the means for atomizing the aerosol source may be vibration atomization or induction heating.

### (2) Second configuration example

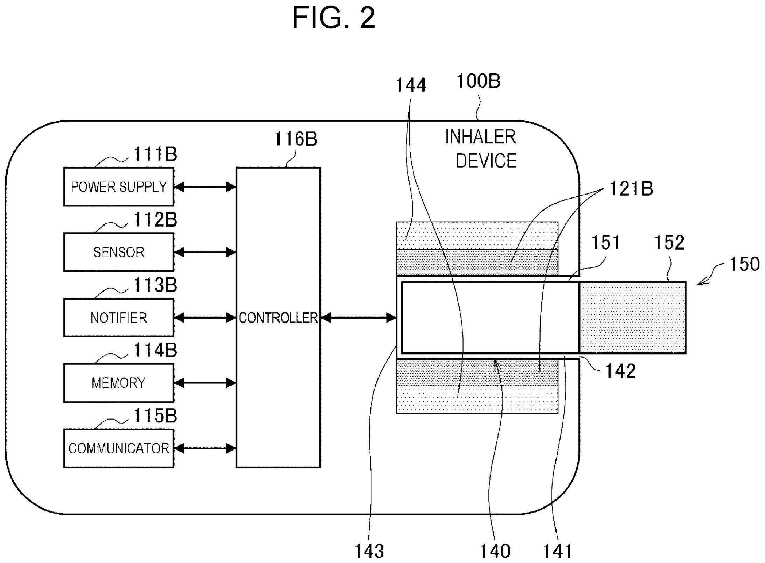

Fig. 2 is a schematic diagram of the inhaler device according to the second configuration example. As illustrated in Fig. 2, an inhaler device 100B according to the present configuration example includes a power supply 111B, a sensor 112B, a notifier 113B, a memory 114B, a communicator 115B, a controller 116B, a heater 121B, a holder 140, and a heat insulator 144.

The power supply 111B, the sensor 112B, the notifier 113B, the memory 114B, the communicator 115B, and the controller 116B are substantially the same as the respective corresponding structural elements included in the inhaler device 100A according to the first configuration example.

The holder 140 has an internal space 141, and holds a stick substrate 150 in a manner partially accommodated in the internal space 141. The holder 140 has an opening 142 that allows the internal space 141 to communicate with outside. The holder 140 holds the stick substrate 150 that is inserted into the internal space 141 through the opening 142. For example, the holder 140 may be a tubular body having the opening 142 and a bottom 143 on its ends, and may define the pillar-shaped internal space 141. The holder 140 can also define a flow path of air to be supplied to the stick substrate 150. For example, the bottom 143 has an air inlet hole that is an inlet of air into the flow path. The opening 142 serves as an air outlet hole that is an outlet of the air from the flow path.

The stick substrate 150 includes a substrate 151 and an inhalation port 152. The substrate 151 includes an aerosol source. The aerosol source according to the present configuration example is not limited to a liquid. The aerosol source may be a solid. The stick substrate 150 held by the holder 140 includes the substrate 151 at least partially accommodated in the internal space 141 and the inhalation port 152 at least partially protruding from the opening 142. When the user inhales with the inhalation port 152 protruding from the opening 142 in his/her mouth, air flows into the internal space 141 through the air inlet hole (not illustrated), and the air and an aerosol generated from the substrate 151 reach inside the mouth of the user.

The heater 121B has a similar configuration to the heater 121A according to the first configuration example. However, in the example illustrated in Fig. 2, the heater 121B has a film-like shape and surrounds the outer circumference of the holder 140. Subsequently, heat produced from the heater 121B heats the substrate 151 of the stick substrate 150 from the outer circumference, generating the aerosol.

The heat insulator 144 prevents heat from transferring from the heater 121B to the other structural elements. For example, the heat insulator 144 may be a vacuum heat insulator or an aerogel heat insulator.

The configuration example of the inhaler device 100B has been described above. The inhaler device 100B is not limited to the above configuration, and may be configured in various ways as exemplified below.

In an example, the heater 121B may have a blade-like shape, and may be disposed so that the heater 121B protrudes from the bottom 143 of the holder 140 toward the internal space 141. In this case, the heater 121B having the blade-like shape is inserted into the substrate 151 of the stick substrate 150 and heats the substrate 151 of the stick substrate 150 from its inside. In another example, the heater 121B may be disposed so that the heater 121B covers the bottom 143 of the holder 140. In still another example, the heater 121B may be implemented as a combination of two or more selected from a first heater that covers the outer circumference of the holder 140, a second heater having the blade-like shape, and a third heater that covers the bottom 143 of the holder 140.

In another example, the holder 140 may include an opening/closing mechanism that at least partially opens and closes an outer shell defining the internal space 141. Examples of the opening/closing mechanism include a hinge. In addition, the holder 140 may sandwich the stick substrate 150 inserted into the internal space 141 by opening and closing the outer shell. In this case, the heater 121B may be at the sandwiching position of the holder 140 and may produce heat while pressing the stick substrate 150.

In addition, means for atomizing the aerosol source is not limited to heating by the heater 121B. For example, the means for atomizing the aerosol source may be induction heating.

In addition, the inhaler device 100B may also include the heater 121A, the liquid guide 122, the liquid storage 123, and the airflow path 180 according to the first configuration example. The air outlet hole 182 of the airflow path 180 may also serve as an air inlet hole to the internal space 141. In this case, a mixture fluid of air and an aerosol generated by the heater 121A flows into the internal space 141, mixes further with an aerosol generated by the heater 121B, and then reaches the oral cavity of the user.

### <<2. Technical problems>>

Patent Literature 1 discloses a technique for detecting user's biological information from a portion of an inhaler device with which a user's finger is in contact.

However, as for a timing of detection of biological information, Patent Literature 1 only mentions that biological information is detected under a predetermined condition. Considering that biological information can have various meanings depending on a timing of detection, it can be said that a technique concerning a detection timing has a room for improvement.

Furthermore, since biological information is detected from the contact portion, biological information cannot be detected unless a user's finger is in contact with a sensor. One measure for detecting biological information is to prompt a user to bring his or her finger into contact with a sensor. However, this requires the user to perform an action unnecessary for a puff and is not appropriate from a perspective of usability.

In order to solve the above problems, a mechanism according to the present embodiment is provided.

### <<Technical features>>

### <3.1 System configuration>

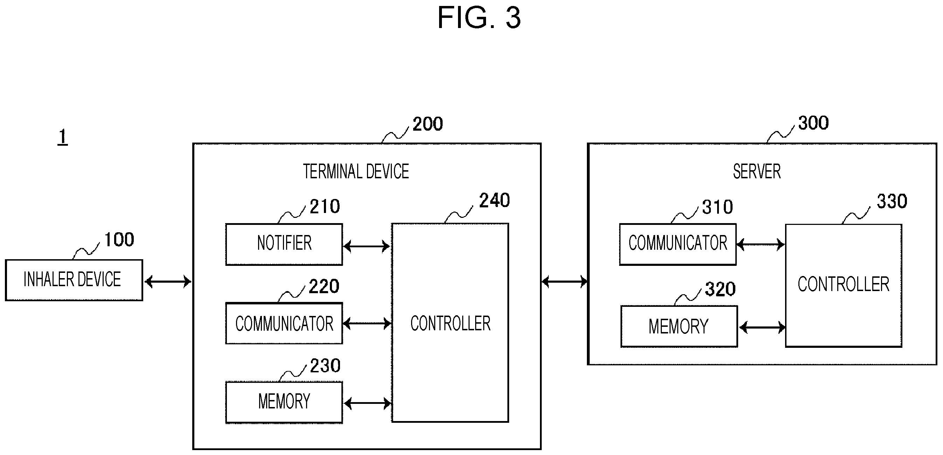

Fig. 3 is a block diagram illustrating an example of a configuration of a system 1 according to an embodiment of the present invention. As illustrated in Fig. 3, the system 1 includes an inhaler device 100, a terminal device 200, and a server 300. The inhaler device 100 and the terminal device 200 are communicable with each other. The terminal device 200 and the server 300 are communicable with each other.

The inhaler device 100 and the terminal device 200 are devices related to each other. For example, the inhaler device 100 and the terminal device 200 are related to each other in that these devices are used by a same user. Alternatively, for example, the inhaler device 100 and the terminal device 200 are related to each other in that these devices are associated with each other, for example, by authentication connection for wireless communication. The authentication connection is means for establishing connection between the inhaler device 100 and the terminal device 200. The authentication connection includes, for example, a process in which the inhaler device 100 and the terminal device 200 exchange their identification information (e.g., service set identifiers (SSIDs)) and a process in which the inhaler device 100 and the terminal device 200 share key information for encrypting/decrypting information to be transmitted or received. Alternatively, the inhaler device 100 and the terminal device 200 are related to each other in that these devices are communicable with each other.

### (1) Inhaler device 100

The inhaler device 100 is a device that generates a material to be inhaled by a user. A user's action of inhaling the material generated by the inhaler device 100 by using the inhaler device 100 is hereinafter referred to simply as inhalation (puff) or an inhaling action. An example of the puff is inhaling with the mouthpiece 124 of the inhaler device 100A according to the first configuration example in a user's mouth. Another example of the puff is inhaling with the inhalation port 152 of the stick substrate 150 inserted into the inhaler device 100B according to the second configuration example in a user's mouth. A user can inhale the material generated by the inhaler device 100 by puffing.

The inhaler device 100 generates the material to be inhaled by a user by consuming content of a substrate. The cartridge 120, the flavor imparting cartridge 130, and the stick substrate 150 described above are an example of the substrate. The aerosol source stored in the liquid storage 123, the flavor source 131 contained in the flavor imparting cartridge 130, and the aerosol source contained in the stick substrate 150 are an example of the content of the substrate. The aerosol is an example of the material to be inhaled by the user.

In the present embodiment, the inhaler device 100 can have any of the first and second configuration examples. That is, the inhaler device 100 according to the present embodiment has a similar configuration to the inhaler device 100A, the inhaler device 100B, or a modification thereof.

Among configurations of the inhaler device 100 according to the present embodiment, supplementary points or emphasized points regarding the configurations of the inhaler device 100A and the inhaler device 100B described in the above configuration examples are mainly described below.

In the first configuration example, the power supply unit 110 and the cartridge 120 are electrically and/or mechanically (including physically) connectable to each other. The power supply unit 110 and the cartridge 120 are attachable and detachable to and from each other. Similarly, the cartridge 120 and the flavor imparting cartridge 130 are electrically and/or mechanically (including physically) connectable to each other. The cartridge 120 and the flavor imparting cartridge 130 are attachable and detachable to and from each other.

Typically, a user inhales in a state where the power supply unit 110 and the cartridge 120 are connected and the cartridge 120 and the flavor imparting cartridge 130 are connected. In a case where the aerosol source contained in the cartridge 120 runs out, the old cartridge 120 is removed and exchanged with a new one. Furthermore, in a case where the flavor component contained in the flavor imparting cartridge 130 runs out, the old flavor imparting cartridge 130 is removed and exchanged with a new one.

### (2) Terminal device 200

The terminal device 200 is a device operated by the user. The terminal device 200 functions as an interface with the user. For example, the terminal device 200 is a smartphone, a tablet terminal, or a wearable device.

As illustrated in Fig. 3, the terminal device 200 includes a notifier 210, a communicator 220, a memory 230, and a controller 240.

The notifier 210 provides information to the user. The notifier 210 includes at least one of a display device that displays information, a light-emitting device that emits light, a vibration device that vibrates, and a sound output device that outputs sound. An example of the display device is a display. An example of the light-emitting device is a light emitting diode (LED). An example of the vibration device is an eccentric motor. An example of the sound output device is a speaker. The notifier 210 provides information to the user by outputting information input from the controller 240. For example, the notifier 210 displays information to be provided to the user, emits light in a pattern of light according to information to be provided to the user, vibrates in a vibration pattern according to information to be provided to the user, or outputs sound or voice of information to be provided to the user.

The communicator 220 is a communication interface for transmitting and receiving information between the terminal device 200 and another device. The communicator 220 performs communication in conformity with any wired or wireless communication standard. Such a communication standard may be, for example, a wireless local area network (LAN), a wired LAN, Wi-Fi (registered trademark), or Bluetooth (registered trademark). Typically, the communicator 220 wirelessly transmits and receives information to and from the inhaler device 100. Furthermore, the communicator 220 transmits and receives information to and from the server 300 over the Internet.

The memory 230 stores various items of information for operation of the terminal device 200. The memory 230 is, for example, a non-volatile storage medium such as a flash memory.

The controller 240 functions as an arithmetic processing unit and a control device, and controls the overall operations of the terminal device 200 in accordance with various programs. The controller 240 includes an electronic circuit such as a central processing unit (CPU) or a microprocessor, for example. In addition, the controller 240 may include a read only memory (ROM) that stores programs and operation parameters to be used and a random access memory (RAM) that temporarily stores parameters that change as appropriate. The terminal device 200 performs various kinds of processing under control of the controller 240. Notification of information by the notifier 210, transmission and reception of information by the communicator 220, storing and readout of information by the memory 230 are examples of processing controlled by the controller 240. Other processing performed by the terminal device 200 such as input of information to the structural elements and processing based on information output from the structural elements is also controlled by the controller 240. Note that the functions of the controller 240 may be realized by an application. The application may be preinstalled or may be downloaded. Alternatively, the functions of the controller 240 may be realized by progressive web apps (PWA).

### (3) Server 300

The server 300 is a device that controls overall service offered to the user of the inhaler device 100. The server 300 is an example of an information processing device of the present invention.

A communicator 310 is a communication interface for transmitting and receiving information between the server 300 and another device. The communicator 310 performs communication in conformity with any wired or wireless communication standard. For example, the communicator 310 communicates with the terminal device 200 over the Internet.

A memory 320 stores various items of information for operation of the server 300. The memory 320 is, for example, a non-volatile storage medium such as a hard disc drive (HDD) or a solid state drive (SSD).

A controller 330 functions as an arithmetic processing unit and a control device, and controls the overall operations of the server 300 in accordance with various programs. The controller 330 includes an electronic circuit such as a central processing unit (CPU) or a microprocessor, for example. In addition, the controller 240 may include a read only memory (ROM) that stores programs and operation parameters to be used and a random access memory (RAM) that temporarily stores parameters that change as appropriate. The server 300 performs various kinds of processing under control of the controller 330. Transmission and reception of information by the communicator 310 and storing and readout of information by the memory 320 are examples of processing controlled by the controller 330. Other processing performed by the server 300 such as input of information to the structural elements and processing based on information output from the structural elements is also controlled by the controller 330.

### <3.2. Technical feature of inhaler device>

### (1) Mechanism for detecting biological information

The inhaler device 100 includes a first detector that detects user's biological information. The first detector is, for example, included in the sensor 112. The biological information is information concerning a body in general. For example, the biological information includes at least one of a blood pressure, a heart rate (or a pulse), a blood oxygen level, and/or a degree of oxygen saturation. The biological information may further include any information such as a body temperature and a breathing rate. The biological information may include information acquired secondarily such as sleepiness obtained by processing information acquired primarily such as a blood pressure. Note that the information acquired secondarily may include mental information such as a user's emotion estimated from physical information such as a blood pressure.

The first detector receives a reflected wave reflected by a user's body and detects biological information on the basis of the received reflected wave. In an example, the reflected wave may be natural light. In this case, the first detector may include a camera, and may detect biological information by analyzing an image taken by the camera. In another example, the first detector may transmit a transmission wave, and the reflected wave may be the transmission wave reflected by a user's body. In this case, the first detector detects biological information on the basis of the reflected wave or a relationship between the reflected wave and the transmission wave. Irrespective of whether or not the transmission wave is transmitted, the first detector can detect user's biological information without contact with the user. For example, the first detector detects information concerning a blood vessel such as a blood pressure on the basis of time-series transition of a diameter of a blood vessel indicated by time-series transition of the reflected wave. The first detector may include an infrared camera and detect a body temperature as a temperature of a skin surface.

In a case where the first detector transmits the transmission wave, the transmission wave includes at least one of a radio wave, an infrared ray, and/or light. Needless to say, the transmission wave may be any other wave as long as biological information can be detected on the basis of a reflected wave that is the transmission wave reflected by a user's body. Note that in a case where the transmission wave is a radio wave, the transmission wave may be, for example, a signal having a bandwidth of at least 3 GHz in a frequency band from 3.0 GHz to 10.7 GHz. By using a signal in such a frequency band as the transmission wave, a reflected wave reflected by a skin surface and a reflected wave reflected by an artery can be separated.

A position where the first detector is provided is not limited. For example, an antenna for transmitting a transmission wave and receiving a reflected wave is provided as the first detector inside a housing of the inhaler device 100.

### (2) Mechanism for detecting information concerning inhaler device 100

The inhaler device 100 includes a second detector that detects information concerning the inhaler device 100. The second detector is, for example, included in the sensor 112. An example of information detected by the second detector is described below.

The second detector may detect input of a user's operation to the inhaler device 100 as the information concerning the inhaler device 100. For example, the second detector may include an input device such as a button or a switch, and detects a user's operation on the input device. In an example, the second detector detects an operation of instructing the inhaler device 100 to start generation of an aerosol.

The second detector may detect user's inhalation, that is, a puff of the aerosol generated by the inhaler device 100 as the information concerning the inhaler device 100. For example, the second detector detects a puff on the basis of a value related to user's inhalation acquired by a pressure sensor such as a condenser microphone, a flow sensor, a temperature sensor, or the like.

The second detector may detect information concerning a substrate used to generate an aerosol by the inhaler device 100 as the information concerning the inhaler device 100. In an example, the second detector detects setting of a substrate in the inhaler device 100. Setting of the substrate in the inhaler device 100 indicates connection of the power supply unit 110, the cartridge 120, and the flavor imparting cartridge 130 in the first configuration example and insertion of the stick substrate 150 into the inhaler device 100B in the second configuration example. In another example, the second detector detects cancellation of setting of the substrate in the inhaler device 100. The cancellation of setting of the substrate indicates cancellation of connection of the power supply unit 110, the cartridge 120, and the flavor imparting cartridge 130 in the first configuration example and removal of the stick substrate 150 from the inhaler device 100B in the second configuration example. In another example, the second detector detects identification information of the substrate set in the inhaler device 100. An example of the identification information of the substrate is information indicative of a type of the substrate. The second detector may include a camera, and may detect setting/cancellation of the substrate on the basis of whether or not the substrate is included in a taken image and may detect identification information of the substrate by analyzing the image including the substrate.

### (3) Timing of detection of biological information

The controller 116 controls the first detector to detect first biological information at a first timing and detect second biological information at a second timing later than the first timing. According to the configuration, the inhaler device 100 can detect biological information at a plurality of different timings. It is therefore possible to determine how an event occurring between the first timing and the second timing influenced a user's body on the basis of a change from the first biological information to the second biological information. An example of the event is a user's puff.

The first timing and the second timing are desirably set so that a timing of a puff is between the first timing and the second timing. Typically, the user often puffs plural times successively, for example, puffs until the aerosol source runs out after a new substrate is set in the inhaler device 100 and heating starts. Such plural puffs performed successively are hereinafter also referred to as successive puffs. The first timing and the second timing are desirably set so that most of a period for which the successive puffs are performed is included between the first timing and the second timing. Furthermore, the first timing and the second timing are desirably set so that a timing of absorption of the aerosol inhaled by the user into a user's body is between the first timing and the second timing. By thus setting the first timing and the second timing, it is possible to determine how a puff influenced the user's body.

In an example, the first timing may be a timing at which the second detector detects input of a predetermined user's operation on the inhaler device 100, a user's action of inhaling (that is, a puff) the aerosol generated by the inhaler device 100, setting of a substrate in the inhaler device 100, or identification information of the substrate set in the inhaler device 100. An example of the predetermined user's operation is an operation for instructing the inhaler device 100 to start generation of the aerosol. Another example of the predetermined user's operation is a user's operation of a predetermined part of the inhaler device 100 for setting of a substrate in the inhaler device 100. The inhaler device 100 may be, for example, provided with an openable and closable cover (not illustrated) that covers the opening 142, and the user may set a substrate in the holder 141 by opening the cover. In this case, the predetermined user's operation may be a user's operation of opening the cover. The timing of detection of identification information of the substrate set in the inhaler device 100 is typically a timing that is the same as or immediately after a timing of setting of the substrate in the inhaler device 100 and is before start of heating. Accordingly, each of these timings is a timing before a puff or a timing at which a puff is performed. Accordingly, the first timing can be set to a timing at least before absorption of the aerosol inhaled by the user into the user's body.

In another example, the first timing may be a timing at which the second detector detects a puff for the first time after a period for which no puff is detected exceeds a predetermined period. The predetermined period is a period longer than a time interval between the successive puffs. In a case where 30 seconds are, for example, assumed as a maximum value of the time interval between the successive puffs, 1 minute is, for example, set as the predetermined period. In this case, an initial puff performed after the period for which no puff is detected exceeds the predetermined period is an initial one of next successive puffs. Therefore, according to the configuration, a timing of an initial one of the successive puffs can be set as the first timing.

The second timing is a timing at least after start of heating of the substrate set in the inhaler device 100. This is because a puff is performed after start of heating of the substrate. According to the configuration, a timing after a puff can be set as the second timing.

In an example, the second timing may be a timing at which cancellation of setting of the substrate in the inhaler device 100 is detected by the second detector. Typically, the user cancels setting of the substrate in the inhaler device 100 and puts the inhaler device 100 into place after finishing tasting flavor. Considering such a user's action, according to the configuration, the second timing can be set to a timing after a puff.

In another example, the second timing may be a timing at which a puff is detected by the second detector last time in a case where a period for which no puff is detected exceeds a predetermined period. In this case, at a timing at which a puff is detected, whether or not this timing is the second timing is unknown, and therefore the first detector detects biological information every time a puff is detected, and uses biological information detected at a timing that meets this condition as the second biological information. A period longer than a time interval between the successive puffs is set as the predetermined period. In a case where 30 seconds are, for example, assumed as a maximum value of the time interval between the successive puffs, 1 minute is, for example, set as the predetermined period. In this case, a puff performed last time in a case where a period for which no puff is detected exceeds the predetermined period is a last one of the successive puffs. Therefore, according to the configuration, a timing at which the last one of the successive puffs is performed can be set as the second timing.

In another example, the second timing may be a timing at which the second detector detects that a predetermined time has elapsed or the number of times of user's inhalation of the aerosol has reached a predetermined number after start of heating of the substrate set in the inhaler device 100. As the predetermined time, a time corresponding to a period for which the successive puffs are performed is set. In a case where 5 minutes are, for example, assumed as the period for which the successive puffs are performed, 4 minutes are, for example, set as the predetermined period. As the predetermined number, a period corresponding to the number of puffs performed as the successive puffs is set. In a case where 15 is, for example, assumed as the number of puffs performed as the successive puffs, 10 is, for example, set as the predetermined number. According to the configuration, the second timing can be set so that most of the period for which the successive puffs are performed is between the first timing and the second timing.

### (4) Processing according to biological information

### - Output of first notification information

The controller 116 controls processing of outputting first notification information, which is information concerning the first biological information and the second biological information.

The first notification information may include information indicative of a difference between the first biological information and the second biological information. The difference may be, for example, a difference in blood pressure, a difference in heart rate, or the like. Alternatively, the difference may be, for example, a secondary difference such as a relax level obtained by processing a primary difference such as a difference in blood pressure or a difference in heart rate. For example, it is estimated that a higher degree of decrease in blood pressure and heart rate indicates a higher relax level. According to the configuration, a receiver of the first notification information can easily recognize how a puff influenced a user's body.

Alternatively, the first notification information may include the first biological information and the second biological information themselves. In this case, a receiver of the first notification information can perform processing for acquiring information indicative of a difference between the first biological information and the second biological information.

The controller 116 determines whether or not to output the first notification information on the basis of the first biological information and the second biological information. Specifically, the controller 116 determines whether or not to output the first notification information by comparing the first biological information and the second biological information. For example, the controller 116 determines that the first notification information is output in a case where a difference between the first biological information and the second biological information meets a predetermined condition and determines that the first notification information is not output in a case where the difference between the first biological information and the second biological information does not meet the predetermined condition. An example of the predetermined condition is that a difference in blood pressure is larger than a predetermined threshold value. According to the configuration, the first notification information can be output in a case where a puff has given predetermined influence to the user.

The controller 116 may control the communicator 115 to transmit the first notification information as controlling processing for outputting the first notification information. For example, the communicator 115 may be a wireless communicator that performs wireless communication and may wirelessly transmit the first notification information. According to the configuration, information indicating how a puff influenced the user's body can be transmitted to another device. An example of a destination is the terminal device 200. The terminal device 200 provides the received information to the user or transmits the received information to the server 300.

The controller 116 may control processing for outputting the first notification information and control the notifier 113 to notify the user of the first notification information. For example, the notifier 113 emits light in a manner corresponding to the first notification information. According to the configuration, the user can be instantly notified of how a puff influenced the user's body.

Note that the first notification information may further include identification information of the substrate used to generate the aerosol in the inhaler device 100. According to the configuration, a receiver of the first notification information can easily recognize which substrate and how the substrate influenced the user's body.

### - Other processing

The controller 116 may limit the function of generating the aerosol on the basis of the first biological information and the second biological information. An example of limiting the function of generating the aerosol is prohibiting heating by the heater 121 for a predetermined time. Another example of limiting the function of generating the aerosol is shifting the inhaler device 100 into a power OFF state. The power OFF state is a state where one or some of the functions of the inhaler device 100 are inexecutable. For example, in the power OFF state, only a function of detecting an action for shifting the inhaler device 100 into an active state (e.g., an operation for instructing the inhaler device 100 to start generation of the aerosol) among the functions of the sensor 112 may be executable. Note that the active state is a state where all of the functions of the inhaler device 100 are executable. For example, the controller 116 determines that the function of generating the aerosol is limited in a case where a difference between the first biological information and the second biological information meets a predetermined condition and determines that the function of generating the aerosol is not limited in a case where a difference between the first biological information and the second biological information does not meet the predetermined condition. An example of the predetermined condition is that a difference in blood pressure is larger than a predetermined threshold value. According to the configuration, the function of generating the aerosol can be limited in a case where a puff has given predetermined influence to the user.

### <3.3 Technical feature of server>

Upon receipt of the first notification information from the inhaler device 100, the terminal device 200 transmits the received first notification information to the server 300.

The communicator 310 receives the first notification information transmitted from the terminal device 200. Then, the memory 320 stores therein the first notification information received by the communicator 310. According to the configuration, information indicating how a puff influenced the user's body can be stored. Furthermore, in a case where the first notification information includes identification information of the substrate used to generate the aerosol, information indicating which substrate and how the substrate influenced the user's body can be stored.

Furthermore, the controller 330 controls processing of outputting the second notification information on the basis of the first notification information received by the communicator 310. For example, the controller 330 controls the communicator 310 to transmit the second notification information in a case where a difference between the first biological information and the second biological information indicated by the received first notification information meets a predetermined condition. An example of the predetermined condition is that a difference in blood pressure is larger than a predetermined threshold value. According to the configuration, the second notification information can be output at an appropriate timing indicated by the user's biological information.

Specifically, the controller 116 controls the communicator 310 to transmit the second notification information as controlling processing for outputting the second notification information. According to the configuration, the second notification information can be transmitted to an appropriate destination.

The destination of the second notification information may be a device associated with another user relevant with the user. An example of the other user is a user's family member, and an example of the device is a smartphone of the user's family member. An example of the second notification information is information indicative of a difference between the first biological information and the second biological information indicated by the received first notification information. According to the configuration, the user's family member can be notified of a user's puff and how the puff influenced the user's body.

The destination of the second notification information may be the inhaler device 100. In this case, the communicator 310 transmits the second notification information to the inhaler device 100 via the terminal device 200. An example of the second notification information is information concerning an instruction to limit the function of generating the aerosol in the inhaler device 100. An example of limiting the function of generating the aerosol is prohibiting heating by the heater 121 for a predetermined time. Upon receipt of the second notification information, the inhaler device 100 limits the function of generating the aerosol. According to the configuration, the function of generating the aerosol in the inhaler device 100 can be limited as needed.

The destination of the second notification information may be the terminal device 200. An example of the second notification information is information obtained by processing a plurality of pieces of first notification information. Specifically, the controller 330 controls processing for outputting information indicative of a substrate recommended for the user as the second notification information on the basis of the first notification information stored in the memory 320. For example, the controller 330 performs statistical processing on a plurality of pieces of first notification information including identification information of a same substrate among accumulated pieces of first notification information and thereby determines influence of the substrate given to the user's body. Then, for example, the controller 330 outputs, as the second notification information, information recommending a substrate that tends to have a high relaxing effect for the user. According to the configuration, a substrate that suits the user's body can be recommended for the user.

### <3.4. Flow of processing>

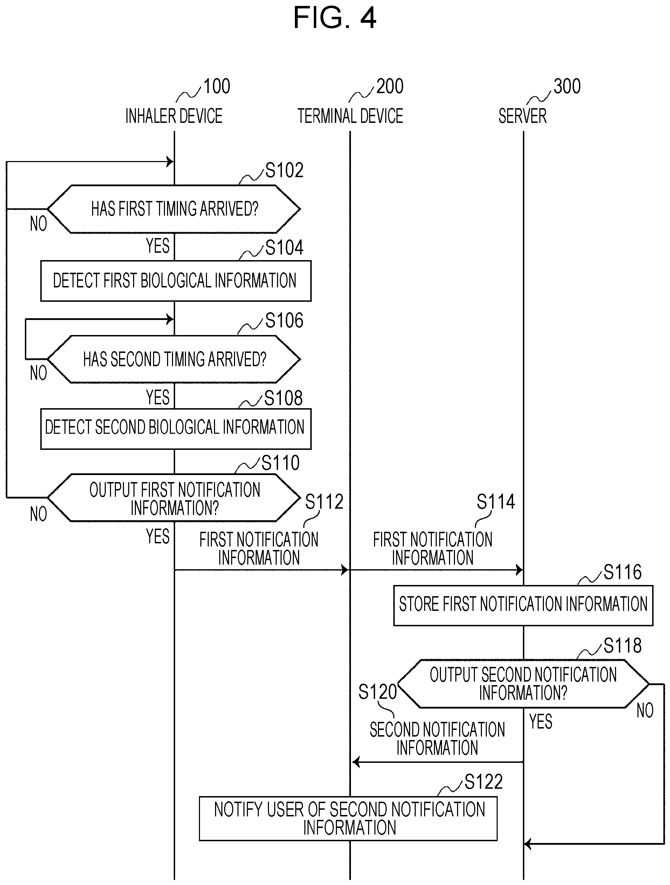

Fig. 4 is a sequence diagram illustrating an example of a flow of processing performed in the system 1 according to the present embodiment. The inhaler device 100, the terminal device 200, and the server 300 are involved in this sequence.

As illustrated in Fig. 4, first, the controller 116 determines whether or not the first timing has arrived (step S102). For example, the controller 116 determines whether or not the first timing has arrived on the basis of information detected by the second detector. In a case where it is determined that the first timing has not arrived (step S102: NO), the controller 116 waits until it is determined that the first timing has arrived. On the other hand, in a case where it is determined that the first timing has arrived (step S102: YES), the first detector detects the first biological information (step S104).

Next, the controller 116 determines whether or not the second timing has arrived (step S106). For example, the controller 116 determines whether or not the second timing has arrived on the basis of information detected by the second detector. In a case where it is determined that the second timing has not arrived (step S106: NO), the controller 116 waits until it is determined that the second timing has arrived. On the other hand, in a case where it is determined that the second timing has arrived (step S106: YES), the first detector detects the second biological information (step S108).

Next, the controller 116 determines whether or not to output the first notification information (step S110) For example, the controller 116 determines that the first notification information is output in a case where a difference between the first biological information and the second biological information meets a predetermined condition, and determines that the first notification information is not output in a case where the difference between the first biological information and the second biological information does not meet the predetermined condition. In a case where it is determined that the first notification information is not output (step S110: NO), the processing returns to step S102. On the other hand, in a case where it is determined that the first notification information is output (step S110: YES), the communicator 115 wirelessly transmits the first notification information (step S112).

Upon receipt of the first notification information, the communicator 220 transmits the received first notification information to the server 300 (step S114)

When the communicator 310 receives the first notification information, the memory 320 stores therein the received first notification information (step S116). Next, the controller 330 determines whether or not to output the second notification information on the basis of the first notification information received by the communicator 310 (step S118). In a case where it is determined that the second notification information is output (step S118: YES), the communicator 310 transmits the second notification information to the terminal device 200 (step S120). On the other hand, in a case where it is determined that the second notification information is not output (step S118: NO), the processing is finished.

When the communicator 220 receives the second notification information, the notifier 210 notifies the user of the second notification information (step S122).

### <<4. Supplements>>

Although a preferred embodiment of the present invention has been described above with reference to the attached drawings, the present invention is not limited to these examples. It is apparent that a person skilled in the art to which the present invention pertains can arrive at various changes or modifications within the technical idea described in the claims, and it should be understood that these changes or modifications are encompassed within the technical scope of the present invention.

For example, although the inhaler device 100 and the server 300 communicate with each other via the terminal device 200 in the above embodiment, the present invention is not limited to this example. The inhaler device 100 and the server 300 may communicate with each other without the terminal device 200. For example, the inhaler device 100 communicates with the server 300 via a base station by using Low Power Wide Area (LPWA). The inhaler device 100 may transmit the first notification information to the server 300 without the terminal device 200. Furthermore, the server 300 may transmit the second notification information to the inhaler device 100 without the terminal device 200.

For example, although the server 300 is an example of an information processing device of the present invention in the above embodiment, the present invention is not limited to this example. For example, the terminal device 200 may function as an information processing device. In this case, the terminal device 200 stores the received first notification information and outputs the second notification information.

Note that the series of processes performed by each device described herein may be realized by using software, hardware, or a combination of software and hardware. Programs that constitute the software are, for example, stored in advance in a recording medium (non-transitory medium) provided inside or outside the device. The programs are, for example, loaded into a RAM when executed by a computer and is executed by a processor such as a CPU. Examples of the recording medium include a magnetic disk, an optical disk, a magneto optical disk, and a flash memory. The computer programs may be, for example, distributed over a network without using a recording medium.

Furthermore, processes described herein by using the flowchart and the sequence diagram need not necessarily be performed in an illustrated order. Some processing steps may be executed in parallel. Furthermore, an additional processing step may be employed or a processing step may be omitted.

### Reference Signs List

- 1: system
- 100: inhaler device
- 110: power supply unit
- 111: power supply
- 112: sensor
- 113: notifier
- 114: memory
- 115: communicator
- 116: controller
- 120: cartridge
- 121: heater
- 122: liquid guide
- 123: liquid storage
- 124: mouthpiece
- 130: flavor imparting cartridge
- 131: flavor source
- 140: holder
- 141: internal space
- 142: opening
- 143: bottom
- 144: heat insulator
- 150: stick substrate
- 151: substrate
- 152: inhalation port
- 180: airflow path
- 181: air inlet hole
- 182: air outlet hole
- 200: terminal device
- 210: notifier
- 220: communicator
- 230: memory
- 240: controller
- 300: server
- 310: communicator
- 320: memory
- 330: controller

## Claims

1. An inhaler device comprising:
a first detector that detects biological information of a user; and
a controller that controls the first detector to detect first biological information at a first timing and detect second biological information at a second timing later than the first timing.

2. The inhaler device according to claim 1, wherein
the first detector receives a reflected wave reflected by a body of the user and detects the biological information on a basis of the received reflected wave.

3. The inhaler device according to claim 2, wherein
the first detector transmits a transmission wave; and
the reflected wave is the transmission wave reflected by the body of the user.

4. The inhaler device according to claim 3, wherein
the transmission wave includes at least one of a radio wave, an infrared ray, and/or light.

5. The inhaler device according to any one of claims 1 to 4, wherein
the controller controls processing for outputting first notification information, which is information concerning the first biological information and the second biological information.

6. The inhaler device according to claim 5, wherein
the controller determines whether or not to output the first notification information on a basis of the first biological information and the second biological information.

7. The inhaler device according to claim 6, wherein
the controller determines whether or not to output the first notification information by comparing the first biological information and the second biological information.

8. The inhaler device according to any one of claims 5 to 7, further comprising a wireless communicator that performs wireless communication with another device,
wherein the controller controls the wireless communicator to transmit the first notification information as controlling processing for outputting the first notification information.

9. The inhaler device according to any one of claims 5 to 8, wherein
the first notification information further includes identification information of a substrate used to generate a material to be inhaled by the user.

10. The inhaler device according to any one of claims 1 to 9, further comprising a second detector that detects information concerning the inhaler device,
wherein the controller controls the first detector to detect the first biological information while using, as the first timing, a timing at which the second detector detects input of a predetermined user's operation to the inhaler device, a user's action of inhaling a material generated by the inhaler device, setting of a substrate for generating the material in the inhaler device, or identification information of the substrate set in the inhaler device.

11. The inhaler device according to claim 10, wherein
the controller controls the first detector to detect the first biological information while using, as the first timing, a timing at which a user's action of inhaling the material is detected by the second detector for a first time after a period for which the user's action of inhaling the material is not detected exceeds a predetermined period.

12. The inhaler device according to claim 10 or 11, wherein
the controller controls the first detector to detect the second biological information while using, as the second timing, a timing after start of heating of the substrate set in the inhaler device.

13. The inhaler device according to claim 10 or 11, wherein
the controller controls the first detector to detect the second biological information while using, as the second timing, a timing at which cancellation of setting of the substrate in the inhaler device is detected by the second detector or a timing at which a user's action of inhaling the material is detected by the second detector last time in a case where a period for which the user's action of inhaling the material is not detected exceeds a predetermined period.

14. The inhaler device according to claim 10 or 11, wherein
the controller controls the first detector to detect the second biological information while using, as the second timing, a timing at which the second detector detects that a predetermined time has elapsed or the number of times of user's inhalation of the material has reached a predetermined number after start of heating of the substrate set in the inhaler device.

15. The inhaler device according to any one of claims 1 to 14, wherein
the controller limits a function of generating a material to be inhaled by the user on a basis of the first biological information and the second biological information.

16. The inhaler device according to any one of claims 1 to 15, wherein
the biological information includes at least one of a blood pressure, a heart rate, a blood oxygen level, and/or a degree of oxygen saturation.

17. An information processing device comprising:
a communicator that communicates with another device; and
a controller that controls processing for outputting second notification information on a basis of first notification information received by the communicator, which is information concerning first biological information detected at a first timing by an inhaler device having a first detector that detects biological information of a user and second biological information detected at a second timing later than the first timing by the inhaler device.

18. The information processing device according to claim 17, wherein
the controller controls the communicator to transmit the second notification information as controlling processing for outputting the second notification information.

19. The information processing device according to claim 18, wherein
the controller controls the communicator to transmit, as the second notification information, information concerning an instruction to limit a function of generating a material to be inhaled by the user in the inhaler device.

20. The information processing device according to any one of claims 17 to 19, wherein
the first notification information further includes identification information of a substrate used to generate a material to be inhaled by the user; and
the information processing device further comprises a memory that stores therein the first notification information.

21. The information processing device according to claim 20, wherein
the controller controls processing for outputting, as the second notification information, information indicative of the substrate recommended for the user on a basis of the first notification information stored in the memory.

22. A control method for controlling an inhaler device including a first detector that detects biological information of a user, the control method comprising:
controlling the first detector to detect first biological information at a first timing and detect second biological information at a second timing later than the first timing.
